# EUROPEAN PATENT SPECIFICATION

(11) **EP 4 444 411 B1**
(45) Date of publication and mention of the grant of the patent: **22.10.2025**
(21) Application number: 22850921.2
(22) Date of filing: 06.12.2022
(51) Int. Cl.: A61N 1/375, A61N 1/372, A61N 1/362, A61N 1/39, A61N 1/36

(54) **IMPLANTABLE MEDICAL DEVICES WITH PULSED FIELD ABLATION**
IMPLANTIERBARE MEDIZINISCHE GERÄTE MIT GEPULSTER FELDABLATION
DISPOSITIFS MÉDICAUX IMPLANTABLES AVEC ABLATION PAR CHAMP PULSÉ

(30) Priority: 07.12.2021 US 202163286606 P
(43) Date of publication of application: 16.10.2024
(73) Proprietor: Medtronic, Inc, Minneapolis, MN 55432 (US)
(72) Inventor: LASKE, Tim, G., Mineapolis, MN 55432 (US); SEIDEL, Rebecca, B., Minneapolis, MN 55432 (US)
(74) Representative: Zimmermann & Partner Patentanwälte mbB
(86) International application number: PCT/US2022/080979
(87) International publication number: WO 2023/107916

(56) References cited:
- WO-A1-2005/058414
- WO-A1-2014/105964
- US-A1- 2009 262 979
- US-A1- 2020 155 863

## Description

### FIELD

This application relates generally to implantable medical devices, and more particularly but not exclusively, to apparatus for treating and managing at least some infections associated with implantable medical devices.

### BACKGROUND

An implantable medical device is placed in the human body during surgery or other clinical intervention to replace a missing biological structure, support a damaged biological structure, or enhance an existing biological structure or function. Implantable medical devices are man-made devices, in contrast to transplants, which are typically natural organs transplanted to a human body from another biological body. Some implantable medical devices contain electrical circuits.

As defined by the U.S. Food and Drug Administration, an active medical device is a "medical device relying for its functioning on a source of electrical energy or any source of power other than that directly generated by the human body or gravity." An implantable medical device is a "medical device which is intended to be totally or partially introduced, surgically or medically, into the human body or by medical intervention into a natural orifice, and which is intended to remain after the procedure." Various active implantable medical devices may remain in the body for several days, weeks, months, or years. US 2020/155863 A1 relates to a method and device for managing pace-assisted high voltage defibrillation shocks. WO 2014/105964 Al relates to systems and methods for delivering pulsed electric fields to skin tissue.

### SUMMARY

Disclosed herein are, among other things, various examples, aspects, features, and embodiments of an active implantable medical device including a pulsed-voltage generator and a plurality of pocket electrodes for creating relatively high voltage gradients in a corresponding body pocket. In some examples, the voltage gradients are greater than approximately 3 kV/cm and are sufficient for killing at least some infectious bacteria in the body pocket via pulsed field ablation. The active implantable medical device further includes an electronic controller that is wirelessly programmable to appropriately control various parameters of the pulsed field ablation, e.g., in a patient- and infection-specific manner. Various examples of the disclosed active implantable medical devices can beneficially be used to provide additional treatment options for infections associated with implantable medical devices.

One example provides an active implantable medical device. The device includes a device box implantable into a body pocket and a plurality of pocket electrodes along an exterior surface of the device box. The device also includes an electrical circuit in an interior portion of the device box, the electrical circuit being electrically connected to the plurality of pocket electrodes to apply thereto voltage pulses producing thereat a voltage gradient of at least 1 kV/cm. The active implantable medical device is selected from the group consisting of a sensor, a gastric pacemaker, a cardiac pacemaker, a cardiac defibrillator, and a stimulator.

Another example provides a medical system. The medical system includes: an active implantable medical device including a first wireless transceiver; a programmer head including a second wireless transceiver, the first wireless transceiver and the second wireless transceiver being configured to wirelessly transmit data therebetween; and an electronic programmer connected to the programmer head. The active implantable medical device includes a device box implantable into a body pocket and a plurality of pocket electrodes along an exterior surface of the device box. The active implantable medical device includes an electrical circuit in an interior portion of the device box, the electrical circuit being electrically connected to the plurality of pocket electrodes to apply thereto voltage pulses producing thereat a voltage gradient of at least 1 kV/cm. The active implantable medical device is selected from the group consisting of a sensor, a gastric pacemaker, a cardiac pacemaker, a cardiac defibrillator, and a stimulator.

### BRIEF DESCRIPTION OF THE DRAWINGS

FIG. 1 is a pictorial diagram illustrating an active implantable medical device according to one example.
FIG. 2 is a block diagram illustrating a medical system used to program and interrogate the active implantable medical device of FIG. 1 according to various examples.
FIG. 3 is block diagram illustrating an electrical circuit used in the implantable medical device of FIG. 1 according to various examples.
FIG. 4 is a schematic diagram illustrating pocket electrodes used in the implantable medical device of FIG. 1 according to some examples.
FIG. 5 is a schematic diagram illustrating pocket electrodes used in the implantable medical device of FIG. 1 according to some additional examples.
FIG. 6 is a block diagram illustrating an exemplary infection-treatment method using the active implantable medical device of FIG. 1 according to various examples.

### DETAILED DESCRIPTION

For illustration purposes and without any implied limitations, example embodiments are described herein below in reference to an implantable cardiac defibrillator (ICD). However, various embodiments are not so limited. Based on the provided description, a person of ordinary skill in the pertinent art will be able to make and use other embodiments without any undue experimentation. In some examples, the corresponding implantable medical device is selected from the group consisting of a sensor, a gastric pacemaker, a cardiac pacemaker, a defibrillator, and a stimulator.

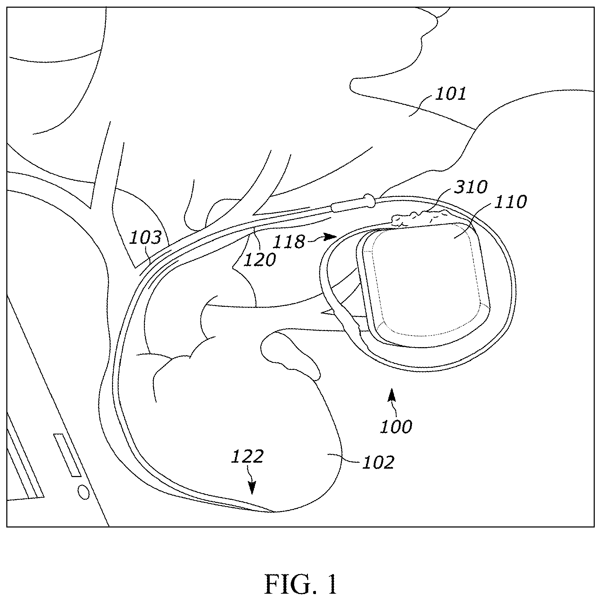

FIG. 1 is a pictorial diagram illustrating an active implantable medical device 100 according to one example. In the example shown, the medical device 100 is an ICD that has been implanted into a living human body 101. Depending on the specific medical condition of the body 101, the ICD 100 can, for example, operate to: (i) correct a cardiac arrhythmia, such as a heart rate or rhythm that is irregular, too fast (tachycardia), or too slow (bradycardia); (ii) prevent sudden cardiac arrest; and (iii) gather data about the function of a heart 102 in the body 101, e.g., to help the corresponding healthcare provider make treatment recommendations. The ICD 100 is different from a pacemaker (which is another example of an active implantable medical device) in that a pacemaker consistently maintains a normal heart rate, whereas the ICD 100 operates to monitor the heart rate and intervene only when needed. However, in some examples, the ICD 100 implements functions of a pacemaker as well.

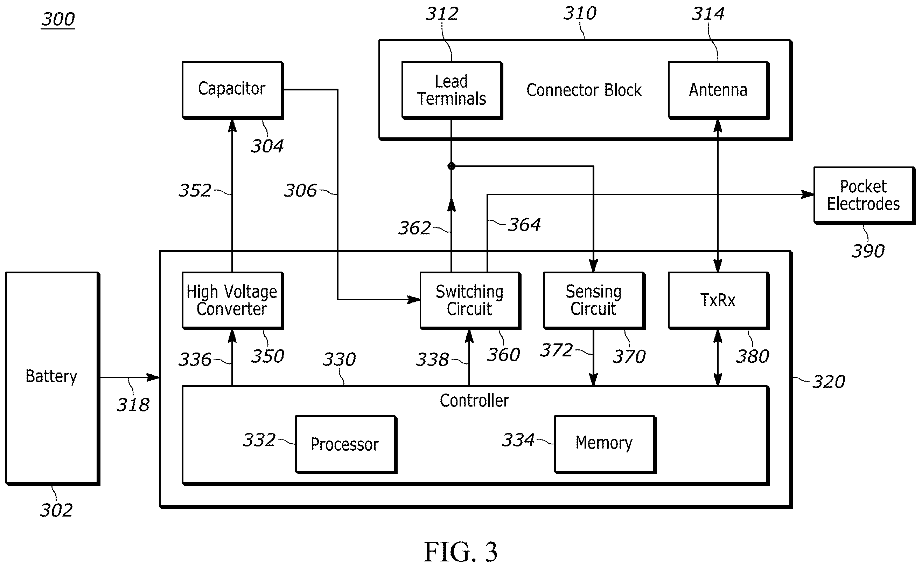

In various examples, the ICD 100 comprises a device box 110 and one, two, or three electrode leads 120. The device box 110 typically includes a pulse generator, a device battery, and additional electronic circuits (not explicitly shown in FIG. 1, e.g., see FIG. 3). The electrode leads 120 typically pass through a vein 103 (usually the large vein under the left or right collarbone) into the heart 102. At a proximal end 118, each of the electrode leads 120 is connected to a respective electrical terminal at the device box 110. At a distal end 122, each of the electrode leads 120 has a respective electrode electrically connected thereto and attached to the heart muscle. The electrode leads 120 are typically made of fine, flexible wires covered with plastic or silicone rubber. The device battery is typically a lithium battery capable of lasting up to approximately 5-7 years under average operating conditions.

In various examples, there are typically two stages to implanting the ICD 100 into the body 101. First, the electrode leads 120 are inserted. Second, the device box 110 is implanted. To insert the electrode leads 120, a surgeon makes a small incision, typically under the left collarbone. The electrode leads 120 are then fed through the vein 103 into the heart 102. When a single electrode lead 120 is inserted, the distal end 122 of the single electrode lead is placed in the right ventricle of the heart 102. When two electrode leads 120 are inserted, the distal end 122 of one is placed in the right ventricle and the distal end 122 of the other is placed in the right atrium of the heart 102. If a third electrode lead 120 is used, then the distal end 122 of the third electrode lead is typically placed in the left ventricle of the heart 102. The position of the electrode lead(s) 120 is typically checked on an X-ray screen. When the electrode leads 120 are in the right place, the electrode leads are secured with one or more stitches. After the electrode leads 120 have been placed and tested, the surgeon makes a small space (typically referred to as a "pocket") for the device box 110 under the muscle or skin, for example, below the left collarbone. The surgeon then connects the proximal ends 118 of the electrode leads 120 to the respective electrical terminals of the device box 110 and places the device box in the pocket. After the requisite testing of certain functions of the ICD 100 in the pocket, the wound is closed.

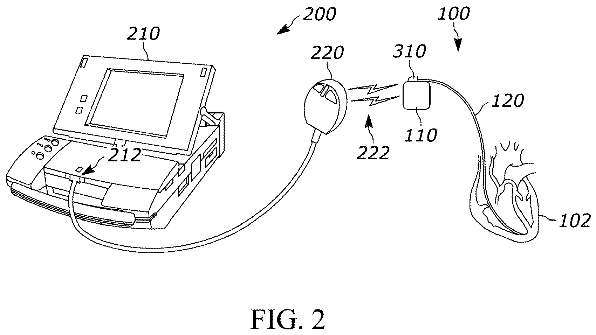

FIG. 2 is a block diagram illustrating a medical system 200 used to program and interrogate the ICD 100 according to various examples. The ICD 100 typically has a set of programmable features. In some examples, the ICD 100 operates to categorize the heart rate as normal, too fast, or too slow by measuring cardiac RR intervals. An RR interval is the time elapsed between two successive R waves of the QRS signal on the electrocardiogram, and its reciprocal is the heart rate. When the ICD 100 detects a threshold number of abnormal RR intervals within a fixed time duration, the internal processor thereof uses a programmed algorithm to decide on the type of intervention, such as anti-tachycardia or anti-bradycardia pacing, synchronized cardioversion, or internal defibrillation. Detailed diagnostic data concerning intracardiac electrograms and event markers are stored in the memory of the ICD 100 and can be retrieved for analyses.

In some examples, programming and interrogation of the ICD 100 are performed using an electronic programmer 210 connected to a programmer head 220. Electronic programmer 210 comprises a computer and an input/output (I/O) interface 212 for connecting the programmer head 220 thereto. The computer runs a program code and communicates with the programmer head 220 via the I/O interface 212. The programmer head 220 includes a wireless transceiver. When the programmer head 220 is placed over a skin portion of the body 101 adjacent to the device box 110 of the ICD 100, the wireless transceiver of the ICD wirelessly sends and receives data-modulated signals 222 to/from a corresponding wireless transceiver located in the device box 110. In various examples, the data-modulated signals 222 are used to monitor the amount of battery life remaining, check and/or change the settings of the ICD 100, ensure that various components of the ICD 100 are functioning properly, download data stored in the memory of the ICD 100 for evaluation, and upload data and program code to the ICD 100 to make software updates and/or configuration changes.

Whenever an implantable medical device, such as the ICD 100, is inserted or implanted into a patient, there is a risk of infection. Bacteria and other pathogens typically associated with such infections can result in serious complications. Some examples of dealing with such infections include the use of antimicrobial coatings or pouches. However, the antimicrobial effect of antimicrobial coatings and pouches attenuates over time and typically becomes too weak to be effective several years after the device implantation. Additionally, in some cases, an infection arises and persists even when the antimicrobial coating or pouch is relatively fresh.

At least some of the above-indicated and possibly some other related problems in the state of the art can beneficially be addressed using various examples, aspects, features, and/or embodiments disclosed herein. Some examples provide an active implantable medical device including a pulsed-voltage generator and a plurality of pocket electrodes for creating strong localized voltage gradients in the corresponding body pocket. The accessible voltage gradient values are in the range between 1 kV/cm and 30 kV/cm, which is typically sufficient for killing at least some infectious bacteria in the body pocket via pulsed field ablation. The active implantable medical device further includes an electronic controller that is wirelessly programmable to appropriately control various parameters of the pulsed-field-ablation procedure, e.g., in a patient- and infection-specific manner. Various examples of the disclosed active implantable medical device can beneficially be used, e.g., to provide additional treatment options for infections associated with implantable medical devices.

FIG. 3 is block diagram illustrating an electrical circuit 300 located at the device box 110 according to various examples. Some parts of the circuit 300 are located inside the device box 110. Some other parts of the circuit 300 are located outside the device box 110, e.g., near or on the exterior surface thereof. In the example shown, the circuit 300 includes a battery 302, a capacitor 304, a connector block 310, a printed circuit board (PCB) 320, and one or more pocket electrodes 390. The battery 302 is connected to provide an electrical power supply 318 to the various circuits located on the PCB 320. The connector block 310 typically protrudes out of the device box 110 and includes lead terminals 312 to which the electrode leads 120 are connected (also see FIGS. 1-2). The connector block 310 also includes an antenna 314 for wirelessly communicating with the programmer head 220 and a suture anchor (not explicitly shown in FIG. 3) for securing the device box 110 in the pocket of the body 101. The battery 302, the capacitor 304, and the PCB 320 are typically located inside the device box 110. The pocket electrodes 390 are located near or on the exterior surface of the device box 110.

The PCB 320 has an electronic controller 330 attached and electrically connected thereto. The electronic controller includes a processor 332, a memory 334, and other circuits for controlling various functions of the circuit 300. In some examples, the processor 332 operates to execute program code having encoded therein various algorithms and instructions for operating the circuit 300. The memory 334 is used to store the program code and further store diagnostic data representing intracardiac electrograms and event markers, configuration and control parameters, device management information, and other data pertinent to the functions of the ICD 100. In some examples, portions of algorithms may be implemented in hardware.

The PCB 320 also has a high-voltage converter 350, a switching circuit 360, a sensing circuit 370, and a wireless transceiver (TxRx) 380 attached and electrically connected thereto. In operation, the high-voltage converter 350 converts a low voltage of the electrical power supply 318 into a high voltage 352 that is used to charge the capacitor 304. The low voltage of the electrical power supply 318 is typically in the range between 2.5 V and 3.3 V. The magnitude of the high voltage 352 is controlled via a control signal 336 generated by the electronic controller 330. In various examples, the high voltage 352 is in the range between 100 V and 10 kV.

The switching circuit 360 operates to generate high-voltage pulses using a high-voltage input 306 received from the capacitor 304. The switching performed in the switching circuit 360 is controlled via a control signal 338 generated by the electronic controller 330. In various examples, the control signal 338 controls the waveform(s) of the generated voltage or current pulses and determines to which of output ports 362 and 364 of the switching circuit 360 the generated pulses are applied. The output port 362 is connected to the lead terminals 312, which are connected to the electrode leads 120 (also see FIGS. 1-2). The output port 364 is connected to the pocket electrodes 390. In various examples, the switching circuit 360 is controlled, via the control signal 338, to generate a pulse waveform characterized by selected values of various parameters. Specific sets of values for such parameters are selected to apply treatment to the heart 102, kill bacteria in the pocket around the device box 110, or stimulate coagulation and healing in the pocket. Various waveform parameters that can be controlled via the control signal 338 include but are not limited to: (i) pulse polarity, e.g., unipolar (monophasic) or bipolar (biphasic); (ii) voltage amplitude; (iii) pulse duration, e.g., from nanoseconds to milliseconds; (iv) interphase interval; (v) inter-pulse interval; (vi) the number of pulses in a pulse sequence; and (vii) the number of pulse sequences in the procedure.

The sensing circuit 370 is connected to the lead terminals 312 to sense electrical (e.g., electrocardiogram) signals delivered thereto via the electrode leads 120. The sensing circuit 370 is electrically isolated from the lead terminals 312 when the high-voltage electrical pulses are being applied thereto by the switching circuit 360. The sensing circuit 370 typically includes one or more sense amplifiers able to respond to varying cardiac signals by changing the sensing threshold, e.g., through an autogain feature thereof, on a fast time scale, e.g., on a beat-to-beat basis. The amplified signals generated by the sense amplifiers are digitized to generate digital signals 372, which are directed to the electronic controller 330 for processing and analysis. In some examples, such processing and analysis are directed at detecting various episodes of cardiac arrhythmia as indicated above.

The wireless transceiver 380 is connected to the antenna 314. The wireless transceiver 380 operates to generate data-modulated radio-frequency (RF) signals based on the data received from the electronic controller 330 and to apply the generated RF signals to the antenna 314 for transmission to the programmer head 220. The wireless transceiver 380 also operates to: (i) receive, through the antenna 314, data-modulated RF signals transmitted by the programmer head 220; (ii) demodulate the received data-modulated RF signals to recover the corresponding data; and (iii) direct the recovered data to the electronic controller 330. In various examples, the wireless transceiver 380 is used to transmit telemetry, send and receive control signals, and perform programming and configuration updates.

The pocket electrodes 390 are configured for pulsed-field ablation directed at killing infectious bacteria in the pocket having the device box 110 therein. Toward this purpose, the pocket electrodes 390 are configured to generate high voltage gradients in the pocket around the device box 110 in response to waveforms applied by the switching circuit 360 to the output port 364. Herein, the term "high voltage gradient" refers to a voltage gradient that is high enough to kill infectious bacteria. Different types of infectious bacteria typically have different respective sensitivities to voltage gradients. Example voltage-gradient values that are sufficient to kill at least some types of infectious bacteria are in the range between about 5 kV/cm and 30 kV/cm. In general, parameters for the waveforms applied by the switching circuit 360 to the output port 364 are selected such that the corresponding voltages and/or voltage gradients kill infectious bacteria while not significantly harming the healthy tissue. In some examples, parameters for the waveforms applied by the switching circuit 360 to the output port 364 are selected to generate local tissue heating to the extent that promotes thermal coagulation and healing. It should also be noted that a voltage gradient is a measure of the strength of the corresponding electric field. In some examples, the terms "voltage gradient" and "electric-field strength" are used interchangeably. In the International System of Units (SI), the electric-field strength is measured in volts per meter (V/m) or newtons per coulomb (N/C). The conversion factor from V/cm to N/C is readily obtained from the following equality: 1 V/cm = 100 N/C.

In one example, a waveform applied by the switching circuit 360 to the output port 364 has the following characteristics: (i) the number of pulses in the corresponding pulse sequence is in the range between 50 and 100; (ii) the generated voltage gradient is between approximately 3 kV/cm and 7 kV/cm; (iii) the pulse-repetition frequency is in the range between 0.1 Hz and 5 Hz; (iv) the peak voltage is ≥3 kV; and (v) the pulse duration is in the range between 10 µs and 100 µs. In another example, a waveform applied by the switching circuit 360 to the output port 364 has the following characteristics: 10 pulses producing a voltage gradient of 15 kV/cm for a duration of 20 µs/pulse with a repetition rate of 1 Hz. In yet another example, the pulse-sequence parameters are: 80 pulses producing a voltage gradient of 5 kV/cm for a duration of 70 µs/pulse with a repetition rate of 0.5 Hz. A person of ordinary skill in the pertinent art will readily understand that other sets of parameter values can also be used depending upon certain characteristics of the patient, the type and severity of infection, and geometric configurations of the pocket electrodes 390.

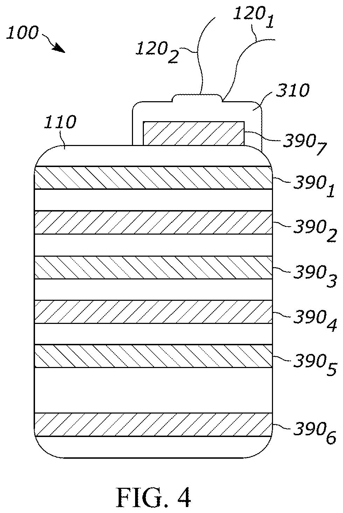

In various examples, the pocket electrodes 390 include an electrode having one of the following geometric shapes/configurations: (i) a flat ribbon electrode; (ii) a paddle electrode; (iii) a braided or woven electrode; (iv) a mesh electrode; (v) a segmented electrode; (vi) a directional electrode; (vii) a patch electrode, and (vii) a coil electrode. In some examples, the pocket electrodes 390 include a pair of interwoven coils. Additional examples of the pocket electrodes 390 are described in more detail below in reference to FIGS. 4-5. In some examples, geometric shapes/configurations for the pocket electrodes 390 are selected based on the type of the corresponding implantable medical device 100, the geometry of the corresponding device box 110, the anatomy of the corresponding pocket, and the type(s) of treatment delivered by the device.

FIG. 4 is a schematic diagram illustrating the pocket electrodes 390 according to some examples. In the example shown, the implantable medical device 100 has two electrode leads 120, which are labeled 120₁ and 120₂, respectively. The pocket electrodes 390 include seven electrodes labeled 390₁-390₇, respectively. The pocket electrodes 390₁-390₆ are metallic (e.g., gold or titanium) stripes located at the exterior surface of the device box 110. The pocket electrode 390₇ is a metallic stripe located at the exterior surface of the connector block 310. In various examples, the pocket electrodes 390₁-390₇ are equidistant or have different interelectrode distances.

In one example, the switching circuit 360 operates to generate a sequence of high-voltage pulses and apply different pulses of the sequence to different respective pairs of the pocket electrodes 390₁-390₇ to generate corresponding voltage gradients therebetween. For example, the first pulse of the sequence is applied between the pocket electrodes 390₁ and 390₂. The second pulse of the sequence is applied between the pocket electrodes 390₂ and 390₃. The third pulse of the sequence is applied between the pocket electrodes 390₂ and 390₃, and so on. Such sequential application of pulses creates a dynamic ablation field that moves around the device box 110.

In another example, the switching circuit 360 has a configuration in which the pocket electrodes 390₁, 390₃, and 390₅ are connected in parallel to one another to form a first multi-stripe electrode, and the pocket electrodes 390₂, 390₄, and 390₆ are similarly connected in parallel to one another to form a second multi-stripe electrode. Each of the pulses of the pulse sequence is applied between the first and second multi-stripe electrodes, thereby generating a static ablation-field pattern around the device box 110.

In some examples, the opposite side of the connector block 310 has another pocket electrode (not directly visible in the shown view) that is similar to the pocket electrode 390₇. In such examples, the switching circuit 360 operates to apply at least some pulses of the sequence of high-voltage pulses between those two electrodes to perform ablation around the connector block 310. In some of such examples, the opposite side of the device box 110 has additional pocket electrodes 390 (not directly visible in the shown view). In different examples, the pattern of the pocket electrodes 390 on the opposite side of the device box 110 is the same as or different from the (shown) pattern of the pocket electrodes 390₁-390₆. The switching circuit 360 further operates to apply at least some pulses of the sequence of high-voltage pulses to the pocket electrodes 390 of the opposite side of the device box 110.

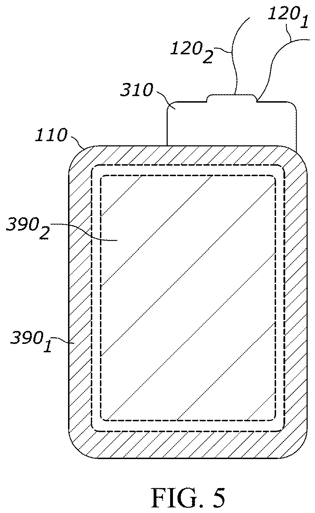

FIG. 5 is a schematic diagram illustrating the pocket electrodes 390 according to some additional examples. In the example shown, the pocket electrodes 390 include two electrodes labeled 390₁ and 390₂, respectively. The pocket electrode 390₁ is laid along the perimeter of the device box 110. The pocket electrode 390₂ is deposited in a middle portion of the side of the device box 110 facing the viewer. The switching circuit 360 operates to generate one or more high-voltage pulses and apply the pulse(s) between the pocket electrodes 390₁ and 390₂ to generate corresponding voltage gradients therebetween. In some examples, the opposite side of the device box 110 has a similar pattern of electrodes 390. In such examples, the switching circuit 360 further operates to apply one or more additional pulses between the pocket electrodes 390 of the opposite side of the device box 110.

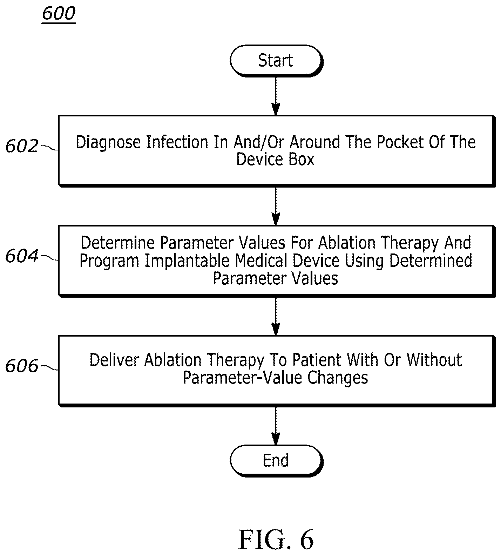

FIG. 6 is a block diagram illustrating an exemplary infection-treatment method 600 according to various examples. The infection-treatment method 600 relies on the ablation therapy capability of the corresponding active implantable medical device having suitable pocket electrodes, such as the pocket electrodes 390 of the ICD 100. The method 600 is described with continued reference to FIGS. 1-5.

The method 600 includes diagnosing an infection in and/or around the pocket of the body 101 into which the device box 110 is implanted (in block 602). One or more diagnostic procedures used in the block 602 are typically performed in a clinical environment during a patient visit. In various examples, such diagnostic procedures include one or more of the following: (i) evaluation of the pocket by a physician; (ii) taking a sonogram of the pocket; (iii) taking an X-ray image of the pocket; (iv) withdrawing a fluid sample from the pocket for analysis; and (v) running a blood test. The one or more diagnostic procedures of the block 602 typically result in identification of the pathogen(s) causing the infection and in infection-treatment recommendations. In some examples, the infection-treatment recommendations include a recommendation for ablation therapy using the pocket electrodes of the corresponding active implantable medical device, such as the pocket electrodes 390 of the ICD 100. Examples of infectious bacteria that can be treated using such ablation therapy include but are not limited to e-coli, staphylococcus epidermidis, staphylococcus capitis, staphylococcus aureus, staphylococcus haemolyticus, and Pseudomonas aeruginosa. When such recommendation for ablation therapy is made, the method 600 is advanced to the operations of block 604.

The method 600 also includes determining a set of parameter values for the ablation therapy (in block 604). In various examples, the parameter values are determined based on the diagnosis of the block 602 and further based on certain characteristics of the patient, such as the estimated tolerance to various forms of ablation therapy. The parameter values determined in the block 604 typically include one or more of: (i) pulse polarity; (ii) voltage amplitude; (iii) pulse duration; (iv) interphase interval; (v) inter-pulse interval; (vi) the number of pulses in a pulse sequence; (vii) allocations of different pulses of the pulse sequence to different respective pairs of the pocket electrodes 390; and (viii) the number of pulse sequences in the procedure. The method 600 further includes programming the corresponding active implantable medical device using the determined parameter values (in block 604). Such programming is typically performed using the corresponding electronic programmer and programmer head, e.g., the electronic programmer 210 and the programmer head 220 for the ICD 100.

The method 600 also includes delivering the ablation therapy to the patient (in block 606). In various examples, such delivery includes further programming the corresponding active implantable medical device to start delivering the waveforms of the ablation therapy at a selected specific time. In such examples, the patient is appropriately prepared for the delivery of the waveforms, typically in the clinical environment and under the supervision of the attending physician. In some examples, some parameter values determined in the block 604 are modified during the delivery, e.g., based on monitoring the vital signs of the patient and/or in response to the subjective reaction of the patient to the delivery of the waveforms.

According to one example disclosed above, e.g., in the summary section and/or in reference to any one or any combination of some or all of FIGS. 1-6, provided is an active implantable medical device, comprising: a device box implantable into a body pocket; a plurality of pocket electrodes along an exterior surface of the device box; and an electrical circuit in an interior portion of the device box, the electrical circuit being electrically connected to the plurality of pocket electrodes to apply thereto voltage pulses producing thereat a voltage gradient of at least 1 kV/cm; and wherein the active implantable medical device is selected from the group consisting of a sensor, a gastric pacemaker, a cardiac pacemaker, a cardiac defibrillator, and a stimulator.

In some examples of the above active implantable medical device, the electrical circuit comprises: a battery to provide a power supply voltage to at least a portion of the electrical circuit; a voltage converter to convert the power supply voltage into a higher voltage, the higher voltage having a magnitude that is at least 50 times larger than a magnitude of the power supply voltage; and a capacitor to be charged to the higher voltage through the voltage converter.

In some examples of any of the above active implantable medical devices, the electrical circuit further comprises a switching circuit configured to apply the higher voltage between a selected pair of the plurality of pocket electrodes.

In some examples of any of the above active implantable medical devices, the electrical circuit further comprises an electronic controller to control the switching circuit to change the selected pair of the plurality of pocket electrodes.

In some examples of any of the above active implantable medical devices, the electrical circuit further comprises an electronic controller to control the switching circuit and the voltage converter to cause the selected pair of the plurality of pocket electrodes to produce, in the body pocket, a voltage gradient greater than 3 kV/cm.

In some examples of any of the above active implantable medical devices, the electrical circuit further comprises a wireless transceiver; and wherein the electronic controller is programable with a program code received via the wireless transceiver, the program code having encoded therein instructions for controlling the switching circuit and the voltage converter.

In some examples of any of the above active implantable medical devices, the switching circuit comprises: a first output port electrically connected to the plurality of pocket electrodes; and a second output port electrically connected to one or more electrical terminals for connecting one or more flexible electrode leads insertable into a body orifice.

In some examples of any of the above active implantable medical devices, the electrical circuit further comprises an electronic controller to control the voltage converter to change the higher voltage.

In some examples of any of the above active implantable medical devices, the higher voltage is at least 3 kV.

In some examples of any of the above active implantable medical devices, the plurality of pocket electrodes includes a plurality of metallic stripes on the exterior surface, different ones of the metallic stripes being electrically isolated from one another.

In some examples of any of the above active implantable medical devices, the active implantable medical device further comprises a connector block protruding through the device box, the connector block including one or more electrical terminals electrically connected to the electrical circuit; and wherein the connector block further includes at least one pocket electrode of the plurality of pocket electrodes.

In some examples of any of the above active implantable medical devices, the plurality of pocket electrodes includes: a first pocket electrode along a perimeter of the device box; and a second pocket electrode in a middle portion of a side of the device box.

According to another example disclosed above, e.g., in the summary section and/or in reference to any one or any combination of some or all of FIGs. 1-6, provided is a medical system, comprising: an active implantable medical device including a first wireless transceiver; a programmer head including a second wireless transceiver, the first wireless transceiver and the second wireless transceiver being configured to wirelessly transmit data therebetween; and an electronic programmer connected to the programmer head; and wherein the active implantable medical device comprises: a device box implantable into a body pocket; a plurality of pocket electrodes along an exterior surface of the device box; and an electrical circuit in an interior portion of the device box, the electrical circuit being electrically connected to the plurality of pocket electrodes to apply thereto voltage pulses producing thereat a voltage gradient of at least 1 kV/cm; and wherein the active implantable medical device is selected from the group consisting of a sensor, a gastric pacemaker, a cardiac pacemaker, a cardiac defibrillator, and a stimulator.

In some examples of the above medical system, the electrical circuit comprises an electronic controller programmable from the electronic programmer using the data transmitted between the first wireless transceiver and the second wireless transceiver.

In some examples of any of the above medical systems, the electrical circuit further comprises: a battery to provide a power supply voltage to at least a portion of the electrical circuit; a voltage converter to convert the power supply voltage into a higher voltage, the higher voltage having a magnitude that is at least 50 times larger than a magnitude of the power supply voltage; a capacitor to be charged to the higher voltage through the voltage converter; and a switching circuit configured to apply the higher voltage between a selected pair of the plurality of pocket electrodes.

In some examples of any of the above medical systems, the electronic controller is programmable to control the switching circuit to change the selected pair of the plurality of pocket electrodes.

In some examples of any of the above medical systems, the electronic controller is programmable to control the switching circuit and the voltage converter to cause the selected pair of the plurality of pocket electrodes to produce, in the body pocket, a voltage gradient greater than 3 kV/cm.

In some examples of any of the above medical systems, the higher voltage is at least 1 kV.

In some examples of any of the above medical systems, the switching circuit comprises: a first output port electrically connected to the plurality of pocket electrodes; and a second output port electrically connected to one or more electrical terminals for connecting one or more flexible electrode leads insertable into a body orifice.

In some examples of any of the above medical systems, the electronic controller is programmable to control the voltage converter to change the higher voltage.

All terms used in the claims are intended to be given their broadest reasonable constructions and their ordinary meanings as understood by those knowledgeable in the technologies described herein unless an explicit indication to the contrary in made herein. In particular, use of the singular articles such as "a," "the," "said," etc. should be read to recite one or more of the indicated elements unless a claim recites an explicit limitation to the contrary.

Unless explicitly stated otherwise, each numerical value and range should be interpreted as being approximate as if the word "about" or "approximately" preceded the value or range.

The use of figure numbers and/or figure reference labels (if any) in the claims is intended to identify one or more possible embodiments of the claimed subject matter in order to facilitate the interpretation of the claims. Such use is not to be construed as necessarily limiting the scope of those claims to the embodiments shown in the corresponding figures.

Reference herein to "one embodiment" or "an embodiment" means that a particular feature, structure, or characteristic described in connection with the embodiment can be included in at least one embodiment of the disclosure. The appearances of the phrase "in one embodiment" in various places in the specification are not necessarily all referring to the same embodiment, nor are separate or alternative embodiments necessarily mutually exclusive of other embodiments. The same applies to the term "implementation."

Unless otherwise specified herein, the use of the ordinal adjectives "first," "second," "third," etc., to refer to an object of a plurality of like objects merely indicates that different instances of such like objects are being referred to, and is not intended to imply that the like objects so referred-to have to be in a corresponding order or sequence, either temporally, spatially, in ranking, or in any other manner.

Unless otherwise specified herein, in addition to its plain meaning, the conjunction "if" may also or alternatively be construed to mean "when" or "upon" or "in response to determining" or "in response to detecting," which construal may depend on the corresponding specific context. For example, the phrase "if it is determined" or "if [a stated condition] is detected" may be construed to mean "upon determining" or "in response to determining" or "upon detecting [the stated condition or event]" or "in response to detecting [the stated condition or event]."

Also, for purposes of this description, the terms "couple," "coupling," "coupled," "connect," "connecting," or "connected" refer to any manner known in the art or later developed in which energy is allowed to be transferred between two or more elements, and the interposition of one or more additional elements is contemplated, although not required. Conversely, the terms "directly coupled," "directly connected," etc., imply the absence of such additional elements. The same type of distinction applies to the use of terms "attached" and "directly attached," as applied to a description of a physical structure. For example, a relatively thin layer of adhesive or other suitable binder can be used to implement such "direct attachment" of the two corresponding components in such physical structure.

The described embodiments are to be considered in all respects as only illustrative and not restrictive. In particular, the scope of the disclosure is indicated by the appended claims rather than by the description and figures herein. All changes that come within the meaning and range of equivalency of the claims are to be embraced within their scope.

The functions of the various elements shown in the figures, including any functional blocks labeled as "processors" and/or "controllers," may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and nonvolatile storage. Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

As used in this application, the term "circuitry" may refer to one or more or all of the following: (a) hardware-only circuit implementations (such as implementations in only analog and/or digital circuitry); (b) combinations of hardware circuits and software, such as (as applicable): (i) a combination of analog and/or digital hardware circuit(s) with software/firmware and (ii) any portions of hardware processor(s) with software (including digital signal processor(s)), software, and memory(ies) that work together to cause an apparatus, such as a mobile phone or server, to perform various functions); and (c) hardware circuit(s) and or processor(s), such as a microprocessor(s) or a portion of a microprocessor(s), that requires software (e.g., firmware) for operation, but the software may not be present when it is not needed for operation." This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in server, a cellular network device, or other computing or network device.

It should be appreciated by those of ordinary skill in the art that any block diagrams herein represent conceptual views of illustrative circuitry embodying the principles of the disclosure. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

## Claims

1. An active implantable medical device, comprising:
a device box (110) implantable into a body pocket;
a plurality of pocket electrodes (390) along an exterior surface of the device box; and
an electrical circuit (300) in an interior portion of the device box, the electrical circuit being electrically connected to the plurality of pocket electrodes to apply thereto voltage pulses producing thereat a voltage gradient of at least 1 kV/cm; and
wherein the active implantable medical device is selected from the group consisting of a sensor, a gastric pacemaker, a cardiac pacemaker, a cardiac defibrillator, and a stimulator.

2. The active implantable medical device of claim 1, wherein the electrical circuit comprises:
a battery (302) to provide a power supply voltage to at least a portion of the electrical circuit;
a voltage converter (350) to convert the power supply voltage into a higher voltage, the higher voltage having a magnitude that is at least 50 times larger than a magnitude of the power supply voltage; and
a capacitor (304) to be charged to the higher voltage through the voltage converter.

3. The active implantable medical device of claim 1 or 2, wherein the electrical circuit further comprises a switching circuit (360) configured to apply the higher voltage between a selected pair of the plurality of pocket electrodes.

4. The active implantable medical device of claim 3, wherein the electrical circuit further comprises an electronic controller (330) to control the switching circuit to change the selected pair of the plurality of pocket electrodes.

5. The active implantable medical device of claim 3, wherein the electrical circuit further comprises an electronic controller (330) to control the switching circuit and the voltage converter to cause the selected pair of the plurality of pocket electrodes to produce, in the body pocket, a voltage gradient greater than 3 kV/cm.

6. The active implantable medical device of claim 5,
wherein the electrical circuit further comprises a wireless transceiver (380); and
wherein the electronic controller is programable with a program code received via the wireless transceiver, the program code having encoded therein instructions for controlling the switching circuit and the voltage converter.

7. The active implantable medical device of claim 3, wherein the switching circuit comprises:
a first output port (364) electrically connected to the plurality of pocket electrodes; and
a second output port (362) electrically connected to one or more electrical terminals for connecting one or more flexible electrode leads insertable into a body orifice.

8. The active implantable medical device of claim 2, wherein the electrical circuit further comprises an electronic controller (330) to control the voltage converter to change the higher voltage.

9. The active implantable medical device of claim 2, wherein the higher voltage is at least 3 kV.

10. The active implantable medical device of claim 1 or 2, wherein the plurality of pocket electrodes includes a plurality of metallic stripes on the exterior surface, different ones of the metallic stripes being electrically isolated from one another.

11. The active implantable medical device of claim 1 or 2, further comprising a connector block (310) protruding through the device box, the connector block including one or more electrical terminals (312) electrically connected to the electrical circuit; and
wherein the connector block further includes at least one pocket electrode of the plurality of pocket electrodes.

12. The active implantable medical device of claim 1 or 2, wherein the plurality of pocket electrodes includes:
a first pocket electrode along a perimeter of the device box; and
a second pocket electrode in a middle portion of a side of the device box.

13. A medical system, comprising:
the active implantable medical device (100) of claim 1, including a first wireless transceiver;
a programmer head (220) including a second wireless transceiver, the first wireless transceiver and the second wireless transceiver being configured to wirelessly transmit data therebetween; and
an electronic programmer (210) connected to the programmer head.

14. The medical system of claim 13, wherein the electrical circuit comprises an electronic controller (330) programmable from the electronic programmer using the data transmitted between the first wireless transceiver and the second wireless transceiver.

15. The medical system of claim 14, wherein the electrical circuit further comprises:
a battery (302) to provide a power supply voltage to at least a portion of the electrical circuit;
a voltage converter (350) to convert the power supply voltage into a higher voltage, the higher voltage having a magnitude that is at least 50 times larger than a magnitude of the power supply voltage;
a capacitor (304) to be charged to the higher voltage through the voltage converter; and
a switching circuit (360) configured to apply the higher voltage between a selected pair of the plurality of pocket electrodes.

## Patentansprüche

1. Aktive implantierbare medizinische Vorrichtung, umfassend:
eine Vorrichtungsbox (110), die in eine Körpertasche implantierbar ist;
eine Mehrzahl von Taschenelektroden (390) entlang einer Außenfläche der Vorrichtungsbox; und
eine elektrische Schaltung (300) in einem inneren Abschnitt der Vorrichtungsbox, wobei die elektrische Schaltung elektrisch mit der Mehrzahl von Taschenelektroden verbunden ist, um Spannungsimpulse daran anzulegen, die daran einen Spannungsgradienten von mindestens 1 kV/cm erzeugen; und
wobei die aktive implantierbare medizinische Vorrichtung aus der Gruppe ausgewählt ist, die aus einem Sensor, einem Magenschrittmacher, einem Herzschrittmacher, einem Herzdefibrillator und einem Stimulator besteht.

2. Aktive implantierbare medizinische Vorrichtung nach Anspruch 1, wobei die elektrische Schaltung Folgendes umfasst:
eine Batterie (302) zum Bereitstellen einer Leistungsversorgungsspannung für mindestens einen Abschnitt der elektrischen Schaltung;
einen Spannungswandler (350) zum Umwandeln der Leistungsversorgungsspannung in eine höhere Spannung, wobei die höhere Spannung eine Größe aufweist, die mindestens 50-mal größer als eine Größe der Leistungsversorgungsspannung ist; und
einen Kondensator (304), der durch den Spannungswandler auf die höhere Spannung aufgeladen werden soll.

3. Aktive implantierbare medizinische Vorrichtung nach Anspruch 1 oder 2, wobei die elektrische Schaltung ferner einen Schaltkreis (360) umfasst, der dazu ausgelegt ist, die höhere Spannung zwischen einem ausgewählten Paar der Mehrzahl von Taschenelektroden anzulegen.

4. Aktive implantierbare medizinische Vorrichtung nach Anspruch 3, wobei die elektrische Schaltung ferner eine elektronische Steuerung (330) umfasst, um den Schaltkreis so zu steuern, dass er das ausgewählte Paar der Mehrzahl von Taschenelektroden wechselt.

5. Aktive implantierbare medizinische Vorrichtung nach Anspruch 3, wobei die elektrische Schaltung ferner eine elektronische Steuerung (330) umfasst, um den Schaltkreis und den Spannungswandler so zu steuern, dass sie das ausgewählte Paar der Mehrzahl von Taschenelektroden veranlassen, in der Körpertasche einen Spannungsgradienten von mehr als 3 kV/cm zu erzeugen.

6. Aktive implantierbare medizinische Vorrichtung nach Anspruch 5,
wobei die elektrische Schaltung ferner einen drahtlosen Sendeempfänger (380) umfasst; und
wobei die elektronische Steuerung mit einem Programmcode programmierbar ist, der über den drahtlosen Sendeempfänger empfangen wird, wobei der Programmcode darin codierte Anweisungen zum Steuern des Schaltkreises und des Spannungswandlers aufweist.

7. Aktive implantierbare medizinische Vorrichtung nach Anspruch 3, wobei der Schaltkreis Folgendes umfasst:
einen ersten Ausgangsport (364), der elektrisch mit der Mehrzahl von Taschenelektroden verbunden ist; und
einen zweiten Ausgangsport (362), der elektrisch mit einem oder mehreren elektrischen Anschlüssen zum Verbinden einer oder mehrerer flexibler Elektrodenleitungen verbunden ist, die in eine Körperöffnung einführbar sind.

8. Aktive implantierbare medizinische Vorrichtung nach Anspruch 2, wobei die elektrische Schaltung ferner eine elektronische Steuerung (330) umfasst, um den Spannungswandler so zu steuern, dass er die höhere Spannung ändert.

9. Aktive implantierbare medizinische Vorrichtung nach Anspruch 2, wobei die höhere Spannung mindestens 3 kV beträgt.

10. Aktive implantierbare medizinische Vorrichtung nach Anspruch 1 oder 2, wobei die Mehrzahl von Taschenelektroden eine Mehrzahl metallischer Streifen auf der Außenfläche aufweist, wobei verschiedene der metallischen Streifen elektrisch voneinander isoliert sind.

11. Aktive implantierbare medizinische Vorrichtung nach Anspruch 1 oder 2, ferner umfassend einen Verbinderblock (310), der durch die Vorrichtungsbox vorsteht, wobei der Verbinderblock einen oder mehrere elektrische Anschlüsse (312) aufweist, die elektrisch mit der elektrischen Schaltung verbunden sind; und
wobei der Verbinderblock ferner mindestens eine Taschenelektrode der Mehrzahl von Taschenelektroden aufweist.

12. Aktive implantierbare medizinische Vorrichtung nach Anspruch 1 oder 2, wobei die Mehrzahl von Taschenelektroden Folgendes aufweist:
eine erste Taschenelektrode entlang eines Umfangs der Vorrichtungsbox; und
eine zweite Taschenelektrode in einem mittleren Abschnitt einer Seite der Vorrichtungsbox.

13. Medizinisches System, umfassend:
die aktive implantierbare medizinische Vorrichtung (100) nach Anspruch 1, die einen ersten drahtlosen Sendeempfänger aufweist;
einen Programmiererkopf (220), der einen zweiten drahtlosen Sendeempfänger aufweist, wobei der erste drahtlose Sendeempfänger und der zweite drahtlose Sendeempfänger dazu ausgelegt sind, drahtlos Daten dazwischen zu übertragen; und
einen elektronischen Programmierer (210), der mit dem Programmiererkopf verbunden ist.

14. Medizinisches System nach Anspruch 13, wobei die elektrische Schaltung eine elektronische Steuerung (330) umfasst, die von dem elektronischen Programmierer unter Verwendung der zwischen dem ersten drahtlosen Sendeempfänger und dem zweiten drahtlosen Sendeempfänger übertragenen Daten programmiert werden kann.

15. Medizinisches System nach Anspruch 14, wobei die elektrische Schaltung ferner Folgendes umfasst:
eine Batterie (302) zum Bereitstellen einer Leistungsversorgungsspannung für mindestens einen Abschnitt der elektrischen Schaltung;
einen Spannungswandler (350) zum Umwandeln der Leistungsversorgungsspannung in eine höhere Spannung, wobei die höhere Spannung eine Größe aufweist, die mindestens 50-mal größer als eine Größe der Leistungsversorgungsspannung ist;
einen Kondensator (304), der durch den Spannungswandler auf die höhere Spannung aufgeladen werden soll; und
einen Schaltkreis (360), der dazu ausgelegt ist, die höhere Spannung zwischen einem ausgewählten Paar der Mehrzahl von Taschenelektroden anzulegen.

## Revendications

1. Dispositif médical implantable actif, comprenant :
un boîtier de dispositif (110) implantable dans une poche corporelle ;
une pluralité d'électrodes de poche (390) le long d'une surface extérieure du boîtier de dispositif ; et
un circuit électrique (300) dans une partie intérieure du boîtier de dispositif, le circuit électrique étant relié électriquement à la pluralité d'électrodes de poche pour leur appliquer des impulsions de tension y produisant un gradient de tension d'au moins 1 kV/cm ; et
le dispositif médical implantable actif étant choisi dans le groupe constitué par un capteur, un stimulateur gastrique, un stimulateur cardiaque, un défibrillateur cardiaque, et un stimulateur.

2. Dispositif médical implantable actif de la revendication 1, dans lequel le circuit électrique comprend :
une batterie (302) pour fournir une tension d'alimentation à au moins une partie du circuit électrique ;
un convertisseur de tension (350) pour convertir la tension d'alimentation en une tension plus élevée, la tension plus élevée ayant une amplitude qui est au moins 50 fois supérieure à une amplitude de la tension d'alimentation ; et
un condensateur (304) devant être chargé jusqu'à la tension plus élevée par le biais du convertisseur de tension.

3. Dispositif médical implantable actif de la revendication 1 ou 2, dans lequel le circuit électrique comprend en outre un circuit de commutation (360) conçu pour appliquer la tension plus élevée entre une paire sélectionnée de la pluralité d'électrodes de poche.

4. Dispositif médical implantable actif de la revendication 3, dans lequel le circuit électrique comprend en outre un dispositif de commande électronique (330) pour commander le circuit de commutation afin de modifier la paire sélectionnée de la pluralité d'électrodes de poche.

5. Dispositif médical implantable actif de la revendication 3, dans lequel le circuit électrique comprend en outre un dispositif de commande électronique (330) pour commander le circuit de commutation et le convertisseur de tension afin d'amener la paire sélectionnée de la pluralité d'électrodes de poche à produire, dans la poche corporelle, un gradient de tension supérieur à 3 kV/cm.

6. Dispositif médical implantable actif de la revendication 5,
dans lequel le circuit électrique comprend en outre un émetteur-récepteur sans fil (380) ; et
dans lequel le dispositif de commande électronique peut être programmé avec un code de programme reçu par le biais de l'émetteur-récepteur sans fil, le code de programme renfermant des instructions codées destinées à commander le circuit de commutation et le convertisseur de tension.

7. Dispositif médical implantable actif de la revendication 3, dans lequel le circuit de commutation comprend :
un premier port de sortie (364) relié électriquement à la pluralité d'électrodes de poche ; et
un deuxième port de sortie (362) relié électriquement à une ou plusieurs bornes électriques afin de relier un ou plusieurs fils d'électrode souples pouvant être insérés dans un orifice corporel.

8. Dispositif médical implantable actif de la revendication 2, dans lequel le circuit électrique comprend en outre un dispositif de commande électronique (330) pour commander le convertisseur de tension afin de modifier la tension plus élevée.

9. Dispositif médical implantable actif de la revendication 2, dans lequel la tension plus élevée est d'au moins 3 kV.

10. Dispositif médical implantable actif de la revendication 1 ou 2, dans lequel la pluralité d'électrodes de poche comporte une pluralité de bandes métalliques sur la surface extérieure, les différentes bandes métalliques étant isolées électriquement les unes des autres.

11. Dispositif médical implantable actif de la revendication 1 ou 2, comprenant en outre un bloc de connexion (310) faisant saillie à travers le boîtier de dispositif, le bloc de connexion comportant une ou plusieurs bornes électriques (312) reliées électriquement au circuit électrique ; et
dans lequel le bloc de connexion comporte en outre au moins une électrode de poche parmi la pluralité d'électrodes de poche.

12. Dispositif médical implantable actif de la revendication 1 ou 2, dans lequel la pluralité d'électrodes de poche inclut :
une première électrode de poche le long d'un périmètre du boîtier de dispositif ; et
une deuxième électrode de poche dans une partie centrale d'un côté du boîtier de dispositif.

13. Système médical, comprenant :
le dispositif médical implantable actif (100) de la revendication 1, comportant un premier émetteur-récepteur sans fil ;
une tête de programmateur (220) comportant un deuxième émetteur-récepteur sans fil, le premier émetteur-récepteur sans fil et le deuxième émetteur-récepteur sans fil étant conçus pour transmettre sans fil des données entre eux ; et
un programmateur électronique (210) relié à la tête de programmateur.

14. Système médical de la revendication 13, dans lequel le circuit électrique comprend un dispositif de commande électronique (330) programmable à partir du programmateur électronique au moyen des données transmises entre le premier émetteur-récepteur sans fil et le deuxième émetteur-récepteur sans fil.

15. Système médical de la revendication 14, dans lequel le circuit électrique comprend en outre :
une batterie (302) pour fournir une tension d'alimentation à au moins une partie du circuit électrique ;
un convertisseur de tension (350) pour convertir la tension d'alimentation en une tension plus élevée, la tension plus élevée ayant une amplitude qui est au moins 50 fois supérieure à une amplitude de la tension d'alimentation ;
un condensateur (304) devant être chargé jusqu'à la tension plus élevée par le biais du convertisseur de tension ; et
un circuit de commutation (360) conçu pour appliquer la tension la plus élevée entre une paire sélectionnée de la pluralité d'électrodes de poche.
